# FASCICULE DE BREVET EUROPEEN

(11) **EP 1 817 620 B1**
(45) Date de publication et mention de la délivrance du brevet: **26.09.2012**
(21) Numéro de dépôt: 05812037.9
(22) Date de dépôt: 21.10.2005
(51) Int. Cl.: G02B 21/00, G02B 21/16

(54) **SYSTEME ET PROCEDE D`IMAGERIE MICROSCOPIQUE MULTIPHOTONIQUE FIBRE D`UN ECHANTILLON**
SYSTEM UND VERFAHREN ZUM AUSFÜHREN EINER GEFASERTEN MEHRPHOTONEN-MIKROSKOPIEBILDGEBUNG EINER PROBE
SYSTEM AND METHOD FOR CARRYING OUT FIBERED MULTIPHOTON MICROSCOPIC IMAGERY OF A SAMPLE

(30) Priorité: 22.10.2004 FR 0411313
(43) Date de publication de la demande: 15.08.2007
(73) Titulaire: Mauna Kea Technologies, 75010 Paris (FR)
(72) Inventeur: VIELLEROBE, Bertrand, 94130 Nogent-sur-Marne (FR); LACOMBE, Francois, 92370 Chaville (FR); LOISEAU, Alexandre, 75009 Paris (FR); LOURADOUR, Frédéric, 87120 Eymoutiers (FR); LELEK, Michaël, 87100 Limoges (FR); BARTHELEMY, Alain, 87000 Limoges (FR)
(74) Mandataire: Pontet, Bernard
(86) Numéro de dépôt international: PCT/FR2005/002630
(87) Numéro de publication internationale: WO 2006/045936

(56) Documents cités:
- US-A- 5 995 281
- US-B1- 6 249 630
- PATENT ABSTRACTS OF JAPAN vol. 2003, no. 12, 5 décembre 2003 (2003-12-05) -& JP 2003 344777 A (JAPAN SCIENCE & TECHNOLOGY CORP), 3 décembre 2003 (2003-12-03)
- WERNER GÖBEL ET AL.: "Distortion-free delivery of nanojoule femtosecond pulses from a Ti:sapphire laser through a hollow-core photonic crystal fiber" OPTICS LETTERS, vol. 29, no. 11, 1 juin 2004 (2004-06-01), pages 1285-1287, XP002329618
- S.W. CLARK ET AL.: "Fiber delivery of femtosecond pulses from a Ti:sapphire laser" OPTICS LETTERS, vol. 26, no. 17, 1 septembre 2001 (2001-09-01), pages 1320-1322, XP002329619 cité dans la demande
- Mankei Tsang ET AL: "Reverse propagation of femtosecond pulses in optical fibers", Optics Letters, vol. 28, no. 20, 15 October 2003 (2003-10-15), page 1873, XP55021692, ISSN: 0146-9592, DOI: 10.1364/OL.28.001873
- GOBEL W ET AL: "Miniaturized two-photon microscope based on a flexible coherent fiber bundle and a gradient-index lens objective", OPTICS LETTERS, OSA, OPTICAL SOCIETY OF AMERICA, WASHINGTON, DC, US, vol. 29, no. 21, 1 November 2004 (2004-11-01), pages 2521-2523, XP002450065, ISSN: 0146-9592, DOI: 10.1364/OL.29.002521
- M. Brecht: "Novel Approaches to Monitor and Manipulate Single Neurons In Vivo", Journal of Neuroscience, vol. 24, no. 42, 20 October 2004 (2004-10-20), pages 9223-9227, XP55021695, ISSN: 0270-6474, DOI: 10.1523/JNEUROSCI.3344-04.2004

## Description

La présente invention se rapporte à un système et un procédé pour réaliser une image microscopique multiphotonique fibrée d'un échantillon, pour une utilisation en endoscopie ou en microscopie de fluorescence. Le domaine d'application visé est plus particulièrement celui de l'imagerie in vivo et in situ.

En imagerie de fluorescence confocale conventionnelle, un photon vient exciter une molécule. La désexcitation de cette dernière provoque le rayonnement d'un photon fluorescent. L'énergie du photon excitateur correspond exactement à la quantité d'énergie nécessaire pour porter la molécule à un état excité donné. La source utilisée est un laser émettant des photons excitateurs dans le visible (entre environ 400nm et 650nm). En microscopie multiphotonique, autrement dit microscopie de fluorescence non-linéaire, et plus particulièrement en microscopie biphotonique, la quantité d'énergie nécessaire à la transition est apportée, non pas par un photon excitateur, mais par deux photons (ou plus en imagerie multiphotonique), chacun présentant une énergie deux fois (ou plus) inférieure à celle du photon excitateur conventionnel. En effet, on utilise des photons excitateurs dans le proche infrarouge (700nm à 1000nm) qui sont moins énergétiques que les photons excitateurs dans le cas conventionnel. Toutefois, le photon fluorescent émis par la molécule est identique à celui émis dans le cas conventionnel.

En microscopie biphotonique (ou multiphotonique) les mécanismes impliquant deux (ou plus) photons ont une efficacité qui est proportionnelle au carré (ou plus) de l'intensité instantanée de la source d'excitation. Une grande efficacité d'excitation ne peut être atteinte que grâce à des contraintes spatiales et temporelles fortes. La contrainte spatiale implique une bonne focalisation du faisceau excitateur dans le tissu, soit une forte densité spatiale des photons dans le volume focal d'éclairement. La microscopie biphotonique présente donc un avantage majeur qui est sa confocalité naturelle puisque toute la fluorescence détectée ne provient que du volume élémentaire excité en profondeur. La fluorescence émise n'est pas une intégrale du volume compris entre la surface de l'échantillon et le volume élémentaire excité; ceci permet notamment de limiter tout problème de photoblanchiment des fluorophores situés entre la surface et le plan de focalisation. La contrainte temporelle implique une source laser générant des impulsions ultra courtes et très intenses, soit une forte densité temporelle des photons dans le volume focal d'éclairement.

Par ailleurs, vis à vis des normes d'éclairement la microscopie biphotonique est appréciable car le proche infrarouge engendre moins d'interaction photons-matière, et une excitation pulsée avec des pulses ultra courts réduit considérablement les problèmes liés à la phototoxicité.

Un inconvénient en microscopie linéaire de fluorescence fibrée réside dans le fait que la distance de pénétration du faisceau excitateur dans l'échantillon est faible, inférieure à une centaine de micromètres. Une augmentation de la puissance de ces faisceaux en vue d'améliorer la distance de pénétration engendrerait certainement des dégâts physiologiques, notamment du fait que l'on est généralement en régime quasi-continu. Ainsi, des organes disposés plus profondément dans l'échantillon ne sont pas accessibles. La microscopie biphotonique permet de palier cet inconvénient puisqu'elle permet une distance de pénétration théorique supérieure à 400 micromètres. En effet, les photons d'excitation, situés dans le proche infrarouge, sont individuellement moins énergétiques, peu absorbés par le tissu qui est essentiellement composé d'eau et donc peu destructeurs en comparaison avec ceux utilisés en fluorescence linéaire.

Les systèmes de microscopie biphotonique couramment utilisés sont des microscopes de table tels que par exemple un microscope droit constitué d'un plateau optique en hauteur portant des dispositifs de balayage et de détection pour la constitution d'images. Un tel système d'acquisition ne peut pas s'appliquer notamment à l'endoscopie in vivo in situ. En effet, un microscope de table est souvent encombrant, utilise des objectifs classiques pour l'illumination et la collection du signal, nécessite le maintien de l'animal sous l'objectif, et nécessite des temps d'intégration longs (garant d'une grande sensibilité).

On connaît le document GB2338568, Optiscan, "Two-photon endoscope or microscope method and apparatus" proposant un dispositif de microscopie biphotonique. Ce dispositif utilise une seule fibre optique pour transporter les impulsions du laser vers l'échantillon. Afin de limiter le phénomène de dispersion linéaire et non linéaire des impulsions dans la fibre optique, des moyens de compensation notamment par prismes sont divulgués.

Le document US6369928, Optical Biopsy Technologies, décrit un microscope à balayage de fluorescence biphotonique pour l'acquisition d'une image microscopique. Ce microscope comporte au moins deux fibres optiques : chacune utilisée comme source et aussi comme récepteur du faisceau de fluorescence obtenu par illumination de l'autre fibre optique. En particulier, deux caractéristiques de ce système constituent des contraintes pour une miniaturisation : 1) le balayage se fait du côté distal des fibres, c'est à dire entre les fibres et l'échantillon; 2) les deux faisceaux incidents respectent un angle d'incidence dans l'échantillon, donc un écartement entre les fibres.

Le document JP 2003 344777 décrit un dispositif d'imagerie comprenant un microscope multiphotonique et un guide d'image constitué d'une pluralité de fibres optiques.

Le document US 6 249 630 B1 décrit un dispositif pour fournir à un appareil optique des pulses optiques, ledit dispositif étant capable de compenser des effets de dispersion.

Le document "Distortion-free delivery of nanojoule femtosecond pulses from a Ti:sapphire laser through a hollow-core photonic crystal fiber", publié dans la revue « Optics Letters »vol.29, N°11, page 1285, décrit une fibre optique non dispersive.

Le document intitulé "Reverse Propagation of femtosecond pulses in optical fibers" de Tsong et al. (optics letters, vol. 28, p. 1873 à 1875) concerne une méthode numérique de calcul de forme de pulse en entrée d'un système optique pour obtenir une autre forme de pulse dérivée en sortie de ce système optique.

La présente invention a pour but un nouveau système de microscopie multiphotonique miniaturisé pour une application en endoscopie notamment. Un autre but de l'invention est un nouveau système de microscopie multiphotonique permettant l'acquisition d'une image en profondeur de l'échantillon.

On atteint au moins l'un des buts précités avec un système d'imagerie multiphotonique fibre selon la revendication 1.

De préférence, pour atteindre des profondeurs importantes, le système comprend une tête optique pour focaliser le faisceau laser d'excitation sortant du guide d'image dans l'échantillon.

De préférence, les dimensions de la tête optique et du guide d'image sont telles qu'ils peuvent aisément se glisser dans un canal opérateur.

Le système selon l'invention permet la réalisation d'une image de fluorescence in vivo, in situ, déportée avec une résolution microscopique. Le guide d'image ou "fiber bundle" en langue anglaise, présente une flexibilité et une taille permettant une application en endoscopie notamment par insertion dans un canal opérateur.

Selon une caractéristique avantageuse de l'invention, différents moyens de compensation peuvent être envisagés, tels que par exemple :
- une fibre optique unique permettant d'optimiser la réponse du guide d'image, cette fibre optique unique étant associée à une ligne dispersive contenant au moins deux prismes ou deux réseaux de diffraction, de telle sorte que les déphasages introduits par cette fibre optique unique et le guide d'image sont compensés par le déphasage introduit par la ligne dispersive;

Ces dispositifs de compensation peuvent également servir à compenser des dispersions introduites par tout autre élément (tête optique, lentilles, miroirs,...) du système.

Selon l'invention, on prévoit des moyens d'injection disposés du côté proximal du guide d'image et permettant de focaliser tour à tour le faisceau laser d'excitation dans une fibre donnée du guide d'image. On prévoit également des premiers moyens de détection pour détecter un signal de fluorescence provenant de l'échantillon. Selon une caractéristique avantageuse, on peut prévoir encore des seconds moyens de détection pour détecter un signal de génération de second harmonique (SHG pour "Second Harmonic Generation" en langue anglaise) provenant de l'échantillon. La complémentarité entre les propriétés de fluorescence biphotonique et de génération de second harmonique permet d'accéder notamment à des informations locales sur les ordres moléculaires (symétrie, organisation) et leur interaction avec leur environnement proche.

Pour que les signaux provenant de l'échantillon atteignent ces moyens de détection, le système comprend en outre un filtre dichroïque adapté pour ne laisser passer que les signaux de fluorescence et de second harmonique vers les détecteurs. En particulier, ce filtre dichroïque est disposé entre les moyens de balayage et le guide d'image. Ainsi, les signaux provenant de l'échantillon ne repassent pas par les moyens de balayage. De la même manière, il n'est pas nécessaire de disposer un trou de filtrage devant chaque détecteur. On tire ici profit du fait que la microscopie multiphotonique présente une confocalité naturelle.

Le système peut également comprendre un filtre dichroïque accordable apte à séparer le signal de fluorescence du signal de génération de second harmonique provenant de l'échantillon.

D'une façon générale, le système comporte une unité de traitement gérant l'ensemble des éléments, notamment la synchronisation entre les moyens d'excitation et les moyens de détection. Cette unité réalise un traitement d'image qui peut par exemple être basé sur celui décrit dans le document WO 2004/008952, Mauna Kea Technologies. On peut par exemple commander un balayage lent de l'échantillon de façon à réaliser des images de grande qualité en intégrant un grand nombre de photons sur un temps long. Toutefois, selon un mode de réalisation avantageux de l'invention, les moyens de balayage balayent l'échantillon à une vitesse correspondant à l'acquisition d'un nombre d'images par seconde suffisant pour une utilisation en temps réel. En complément, les moyens de détection détectent le signal de fluorescence à une fréquence de détection correspondant à une fréquence minimale d'échantillonnage des fibres une à une. Plus précisément, le respect de l'échantillonnage des fibres (selon le critère de Shannon) permet d'obtenir une image point à point correspondant bien à chaque fibre. Cela permet de ne pas perdre d'information en échantillonnant l'ensemble des fibres une à une tout en respectant un nombre moyen minimal d'images par seconde, à savoir en pratique au minimum 12 images par secondes pour un mode maximal de 896 x 640 pixels. Le choix de la fréquence de détection (bande passante du détecteur) en fonction de cet échantillonnage minimal permet ensuite pour chaque fibre de détecter le plus grand nombre possible de photons de fluorescence. Ainsi, selon un mode de réalisation possible, mettant en oeuvre un guide d'image d'environ 30000 fibres optiques souples, la fréquence d'échantillonnage et la bande passante du système de détection (une photodiode à avalanche ou équivalent) sont fixés sensiblement à 1,5 MHz, correspondant environ à 12 pixels par fibre, permettant alors d'obtenir au minimum les 12 images/s en mode maximal 896 x 640 pixels. En pratique, la déviation du faisceau est réglée en déterminant une fréquence de balayage rapide d'un miroir résonnant "ligne" et une fréquence de balayage lente d'un miroir galvanométrique "trame". Ceci permet un balayage rapide approprié des fibres pour obtenir une image en temps réel.

Les miroirs galvanométriques peuvent aussi avoir des fréquences de balayage adaptées pour une acquisition lente; dans ce cas, le photodétecteur présente une bande passante adaptée à la vitesse d'acquisition lente.

Selon l'invention, les moyens de balayage peuvent également balayer l'échantillon sur une ligne dans un plan subsurfacique de façon à réaliser un balayage linéaire ("linescanning" en langue anglaise). On peut ainsi mesurer des intensités ou des vitesses de certains éléments observés.

Le laser pulsé peut être un laser femtoseconde ou un laser picoseconde. Le choix d'un type de laser dépend du type (en terme de sensibilité notamment) de fluorescence ciblé. Par exemple, un laser picoseconde présente des pulses plus longs, donc un laser utile a priori pour des fluorophores à haut rendement. En fait, on peut par exemple utiliser des largeurs de pulse comprises entre 10 picosecondes et 10 femtosecondes.

Par ailleurs, selon l'invention, le laser pulsé et les moyens de compensation sont accordables en longueur d'onde. On peut alors utiliser un laser dont la longueur d'onde peut varier entre 700nm et 1000nm, de préférence entre 800nm et 870nm, ce qui permet déjà de détecter un grand nombre de fluorophores. A chaque longueur d'onde du laser, on adapte la compensation.

Selon un autre aspect de l'invention, il est proposé un procédé d'imagerie multiphotonique fibré selon la revendication 18. .

Avantageusement, on détecte l'ensemble du signal de fluorescence sortant du guide d'image. Ainsi on ne dé-balaye pas et on ne filtre pas le signal de fluorescence avant détection par un photodétecteur.

Dans une variante de l'invention sans tête optique, le guide d'image est constitué de plusieurs milliers de fibres optiques dont les extrémités distales sont destinées à être placées à nue directement en contact de la surface de l'échantillon, chaque fibre étant adaptée à produire un faisceau divergent susceptible d'exciter un micro-volume de l'échantillon situé de la surface jusqu'à une profondeur maximale dépendante notamment du diamètre de coeur des fibres optiques. Sur les dix premiers micromètres par exemple, le faisceau présente encore un diamètre qui est sensiblement identique au diamètre du coeur de la fibre optique. Pour un guide d'image appelé sonde "S", le diamètre des fibres optiques utilisées est suffisamment faible, par exemple 1 micromètre, pour que le phénomène multiphotonique apparaisse.

Cette variante se différentie donc de la variante avec tête optique en ce qu'elle ne prévoit pas le balayage d'un signal qui est focalisé en sortie de chaque fibre mais le balayage d'un signal divergent en sortie de chaque fibre. La non focalisation du signal en sortie de fibre permet d'obtenir des images d'un volume situé juste sous la surface du tissu qui sont exploitables et intéressantes d'un point de vue médical notamment. Ces images ne sont pas "confocales" car elles ne proviennent pas d'un plan de coupe subsurfacique balayé point à point, mais des images que l'on peut toutefois qualifier de "hautement résolues" car provenant du balayage tour à tour de microvolumes situés directement sous la surface.

Un des avantages d'une telle variante réside dans le fait que pour une application endoscopique, le diamètre de la sonde endoscopique peut être très petit dépendant uniquement du diamètre du guide d'image et donc de son nombre de fibres optiques. Cela permet d'envisager des domaines d'applications, comme par exemple le domaine de la neurologie, où la taille de la sonde endoscopique est un facteur critique en s'affranchissant des problèmes inhérents à la miniaturisation de la tête optique de focalisation.

Le système peut comprendre des moyens de filtrage et de détection pour respectivement dissocier et détecter plusieurs signaux de fluorescence émis par plusieurs fluorophores qui sont présents dans l'échantillon et qui sont excités par le faisceau laser d'excitation. En effet, idéalement on génère un faisceau laser pulsé dont la longueur d'onde a été déterminée pour exciter un fluorophore donné. Cependant, d'autres fluorophores peuvent être sensibles à cette longueur d'onde, et émettre alors également des signaux de fluorescence. On peut aussi délibérément introduire des fluorophores et utiliser une longueur d'onde du faisceau laser susceptible d'exciter simultanément ces fluorophores. De préférence, les signaux de fluorescence présentent des longueurs d'onde suffisamment éloignées les unes des autres pour qu'ils soient dissociables par filtrage. Le système comprend des moyens de traitement pour élaborer une image finale comprenant des zones colorées en fonction des signaux de fluorescence des fluorophores. Le système est donc adapté pour réaliser du multimarquage par voie de détection. Les moyens de filtrage peuvent comprendre un filtre passe-bande accordable laissant passer séquentiellement les différents signaux de fluorescence vers un détecteur commun. Les moyens de filtrage peuvent aussi comprendre un séparateur apte à envoyer, en fonction de la longueur d'onde, chaque signal de fluorescence vers un détecteur différent.

Selon encore une autre caractéristique avantageuse, le système peut comprendre en outre un spectromètre apte à élaborer un spectre à partir d'une partie du signal provenant de l'échantillon. Ce spectromètre peut être associé à un obturateur dirigeant une partie du signal provenant de l'échantillon vers le spectromètre à des instants prédéterminés correspondant aux instants où le signal d'excitation balaye une zone d'intérêt. Autrement, on peut aussi commander le laser pulsé de façon à ce que seules des zones d'intérêts soient illuminées. Le spectre élaboré est ensuite traité au sein des moyens de traitement. La partie du signal déviée vers le spectromètre est de préférence inférieure à 10% du signal utile.

D'autres avantages et caractéristiques de l'invention apparaîtront à l'examen de la description détaillée d'un mode de mise en oeuvre nullement limitatif, et des dessins annexés, sur lesquels :
- La figure 1 est une vue schématique du système d'acquisition selon l'invention;
- La figure 2 est une vue schématique de dessus d'un système doté d'une compensation à base de réseaux de diffraction;
- La figure 3 est une vue schématique de dessus d'un système doté d'une compensation à base de prismes;
- La figure 4 est une vue schématique de dessus d'un système doté d'une compensation à base de quatre réseaux de diffraction;
- La figure 5 est une vue schématique de dessus d'un système doté d'une compensation à base de quatre prismes;
- La figure 6 illustre un système équivalent à celui décrit sur la figure 2 avec en plus un masque de phase et un masque d'amplitude agissant sur la forme de l'impulsion en phase et en amplitude;
- La figure 7 illustre un système équivalent à celui décrit sur la figure 3 avec en plus un masque de phase et un masque d'amplitude agissant sur la forme de l'impulsion en phase et en amplitude;
- La figure 8 illustre un système selon l'invention équivalent à celui décrit sur la figure 2 avec en plus un tronçon dispersif de fibre optique;
- La figure 9 illustre un système selon l'invention équivalent à celui décrit sur la figure 3 avec en plus un tronçon dispersif de fibre optique;
- La figure 10 illustre un système selon l'invention équivalent à celui décrit sur la figure 4 avec en plus un tronçon dispersif de fibre optique ;
- La figure 11 illustre un système selon l'invention équivalent à celui décrit sur la figure 5 avec en plus un tronçon dispersif de fibre optique ;
- La figure 12 est une vue schématique de dessus d'un système doté d'une compensation à base de fibre optique à dispersion anormale;
- La figure 13 est une vue de dessus schématique d'un système dans lequel le système de balayage et le dispositif de compensation sont combinés; et
- La figure 14 illustre un système selon l'invention équivalent à celui décrit sur la figure 13 avec en plus un tronçon dispersif de fibre optique .

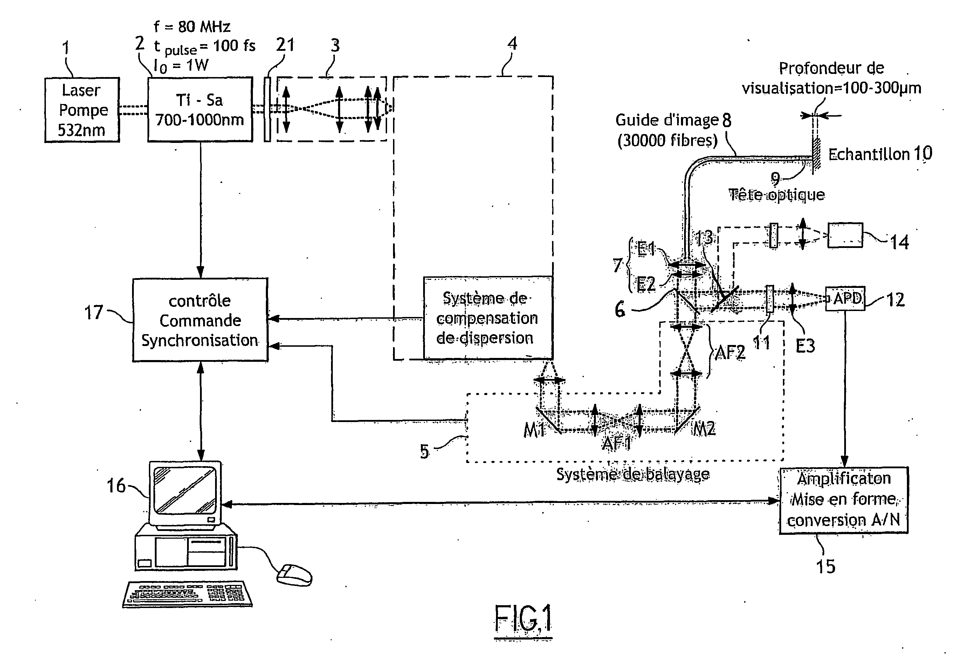

Sur la figure 1 on distingue un échantillon 10 qui peut être un tissu biologique ou une culture cellulaire. En général, la fluorescence observée peut provenir d'un composé exogène (typiquement un marqueur administré) ou d'un composé endogène qui est soit fabriquée par des cellules (marqueur de type transgénique) d'un tissu biologique, soit naturellement présent dans les cellules (autofluorescence).

L'absorption non linéaire à deux photons nécessite d'amener une très forte densité d'énergie dans un volume réduit. Pour ce faire, on utilise un laser pulsé 2 en régime femtoseconde avec des largeurs de pulses de 100fs. Il s'agit d'un laser Titane-Saphir pompé par un laser solide 1 à 532 nm. La cadence de répétition du laser 2 est d'environ 80 MHz avec une puissance moyenne de l'ordre de 1 Watt. La longueur d'onde du faisceau d'excitation sortant du laser 2 peut être réglée entre 700 et 1000 nm, proche infrarouge, de préférence entre 800nm et 870nm. En fait, les performances du système dépendent essentiellement des caractéristiques de la source : puissance crête et largeur des pulses souhaitées notamment en sortie du guide d'image.

En sortie du laser 2, est placé un isolateur de Faraday 21 pour éviter que des réflexions parasites retournent vers la cavité du laser 2. L'isolateur 21 est éventuellement suivi, lorsque nécessaire, d'un dispositif 3 de mise en forme et d'injection du faisceau laser d'excitation. Ce dispositif 3 est constitué d'un système optique afocal de grandissement différent de 1, composé de lentilles qui permettent de modifier le diamètre du faisceau laser. Le grandissement est calculé de sorte que le diamètre du faisceau laser est adapté à des moyens d'injection prévus pour diriger ce faisceau laser dans des moyens de compensation 4. Ces moyens de compensation sont réglés en position et en angle en fonction de la longueur d'onde du faisceau d'excitation.

D'une façon générale, les moyens de compensation 4 ont pour fonction de pré-compenser l'élargissement des impulsions d'excitation dans les fibres optiques du guide d'image 8. Cet élargissement temporel est dû à la dispersion chromatique linéaire et aux effets non linéaires des fibres optiques (auto-modulation de phase provoquant un élargissement spectral). Le système permet d'obtenir une largeur de pulse en sortie du guide d'image 8 de quelques centaines de femtoseconde avec une puissance moyenne de quelques dizaines de milliwatt.

Des moyens de balayage 5 récupèrent ensuite les impulsions d'excitation ainsi pré-compensées. Selon l'exemple choisi et représenté sur la figure 1, ces moyens comprennent un miroir M1 résonant à 4 KHz servant à dévier le faisceau horizontalement et donc à réaliser les lignes de l'image, d'un miroir M2 galvanométrique à 15 Hz, en général entre 10 et 40 Hz, servant à dévier le faisceau verticalement et donc à réaliser la trame de l'image ; et de deux systèmes afocaux de grandissement unitaire, AF1 situé entre les deux miroirs et AF2 situé après le miroir M2, ces systèmes afocaux étant utilisés pour conjuguer les plans de rotation des deux miroirs M1 et M2 avec le plan d'injection dans l'une des fibres. Selon l'invention, la vitesse de balayage est déterminée pour permettre une observation des tissus in vivo in situ. Pour cela, le balayage doit être suffisamment rapide pour qu'il y ait au moins 12 images/s affichées à l'écran pour un mode d'affichage de 896 x 640 pixels correspondant au mode le plus lent. Pour les modes d'affichage ayant moins de pixels, le nombre d'images acquises par seconde sera ainsi toujours supérieur à 12 images/s. En variante, les moyens de balayage peuvent comprendre notamment un miroir rotatif, des composants intégrés de type MEMs (miroirs de balayage X et Y), ou un système acousto-optlque.

Les miroirs M1 et M2 peuvent aussi être deux miroirs galvanométriques dont les fréquences de balayage sont telles qu'on utilise moins de dix images par seconde, par exemple 1 à 3 images par seconde. Dans ce cas, la bande passante du photodétecteur associé est adaptée à la vitesse d'acquisition imposée par les miroirs galvanométriques. Le temps d'intégration peut être long de façon à augmenter la sensibilité du système.

Le faisceau d'excitation dévié en sortie des moyens de balayage 5 est dirigé vers les moyens optiques 7 afin d'être injecté dans l'une des fibres du guide d'image 8. Le filtre dichroïque 6 disposé entre les moyens de balayage 5 et les moyens d'injection 7 reste transparent au faisceau d'excitation. Les moyens d'injection 7 sont constitués ici de deux ensembles optiques E1 et E2. Le premier ensemble optique E1 permet de corriger en partie les aberrations optiques en bord de champ des moyens de balayage 5, l'injection étant ainsi optimisée sur l'ensemble du champ optique, au centre comme au bord. Le second ensemble optique E2 est destiné à réaliser l'injection proprement dite. Sa focale et son ouverture numérique ont été choisies pour optimiser le taux d'injection dans les fibres optiques du guide 8. Selon un mode de réalisation permettant d'obtenir le critère d'achromaticité, le premier ensemble E1 est constitué d'un doublet de lentilles, et le second ensemble E2 de deux doublets de lentilles suivi d'une lentille située près du guide d'image. En variante, cette optique d'injection pourrait être constituée de tout autre type d'optiques standards, comme par exemple deux triplets, ou de lentilles à gradient d'indice (avec une correction du chromatisme par des éléments optiques diffractifs) ou bien d'un objectif de microscope.

Le guide d'image 8 est constitué d'un très grand nombre de fibres optiques souples, par exemple 30000 fibres en silice dopé germanium, chacune monomode, de 2 µm de diamètre, d'ouverture numérique de 0,23 et espacée de 3,8 µm par rapport à sa voisine. La section transversale du guide est de l'ordre de 0,8 mm. En pratique, on peut utiliser soit l'ensemble des fibres du guide d'image, soit un sous-ensemble choisi de ces fibres, par exemple centré. En variante, le guide d'image peut comporter des fibres multimodes de 1,9 µm de diamètre, d'ouverture numérique de 0,42 et espacée de 3,3 µm pour une section transversale du guide de l'ordre de 0,65 mm.

L'extrémité distale de la fibre optique est connectée à une tête optique 9 qui focalise le faisceau laser d'excitation dans l'échantillon 10 en un volume élémentaire. Ce volume ou point élémentaire est situé à une profondeur donnée se trouvant à quelques centaines de µm de la surface de l'échantillon au contact de laquelle est destinée à être placée la tête optique 9. Cette profondeur peut être par exemple de 200 µm. La tête optique 9 permet donc de focaliser le flux sortant du guide d'image dans l'échantillon, mais également de collecter le flux de fluorescence revenant de l'échantillon. La tête optique 9 possède un grandissement de 2,4 et une ouverture numérique sur l'échantillon de 0,5. La microscopie biphotonique présentant naturellement un caractère confocal, il n'est pas nécessaire de filtrer le signal de fluorescence capté par le photodétecteur : tous les différents flux de ce signal sont envoyés vers le photodétecteur, ce qui améliore la sensibilité du système. Avec ces valeurs de grandissement et d'ouverture numérique, la résolution axiale est de l'ordre de 15 µm et la résolution latérale de l'ordre de 2 µm. L'ouverture numérique est également choisie de manière à optimiser le nombre de photons récupérés qui doit être le plus important possible. La tête optique peut être constituée d'optiques classiques (doublet, triplet, asphérique) et/ou de lentilles à gradient d'indice (GRIN). En fonctionnement, la tête optique est notamment destinée à être posée au contact de l'échantillon 10. De manière optimale, la tête optique comporte des optiques réfractives avec un grandissement de 4 et une ouverture numérique de 1. Cette tête optique est de type à immersion dans l'eau et non achromatique.

Le signal de fluorescence traverse donc le guide d'image 8, les moyens d'injection 7 puis réfléchit sur le filtre dichroïque 6 qui dirige ce signal de fluorescence vers un détecteur de fluorescence 12 via un filtre 11 de réjection coloré et une lentille de focalisation E3.

Le filtre dichroïque 6 possède une efficacité de transmission de 98 à 99 % à la longueur d'onde d'excitation et réfléchit donc les autres longueurs d'onde. Le signal de fluorescence, en provenance de l'échantillon via la tête optique et le guide d'image, est ainsi envoyé vers la voie de détection. Le filtre de réjection 11 permet d'éliminer totalement les 1 à 2 % de réflexions parasites à la longueur d'onde d'excitation et qui passent tout de même vers la voie de détection.

Le détecteur 12 présente une sensibilité maximale à la longueur d'onde de fluorescence étudiée. On peut utiliser par exemple une photodiode à avalanches (APD) ou bien un photomultiplicateur. Par ailleurs, selon l'invention, la bande passante est choisie pour optimiser le temps d'intégration du signal de fluorescence. Elle est de 1,5 MHz en temps réel, ce qui correspond à la fréquence d'échantillonnage minimale du guide d'image avec un temps d'intégration optimisé sur chaque pixel.

Le système selon la présente invention est notamment remarquable par le fait qu'il permet d'associer à la microscopie multiphotonique la microscopie de génération de second harmonique. Il s'agit de détecter le signal de génération de second harmonique émis en même temps que le signal de fluorescence par l'échantillon. Pour ce faire, on prévoit un filtre dichroïque accordable 13 ou tout autre dispositif placé entre le filtre dichroïque 6 et le filtre de réjection 11, et permettant de séparer le signal de génération de second harmonique du signal de fluorescence. Un détecteur SHG 14 recueille ce signal de génération de second harmonique.

Les moyens électroniques et informatiques 16 (tel qu'un micro-ordinateur) de commande, d'analyse et de traitement numérique du signal détecté et de visualisation comprennent les cartes suivantes :
- une carte de synchronisation 17 qui a pour fonctions:
- de commander de manière synchronisée le balayage, c'est-à-dire le mouvement des miroirs ligne M1 et trame M2 ;
- de commander de manière synchronisée avec les images de fluorescence, l'analyse des données en provenance du détecteur SHG 14;
- de connaître à tout instant la position du spot laser ainsi balayé ;
- de gérer toutes les autres cartes par l'intermédiaire d'un microcontrôleur lui-même pouvant être piloté ; et
- de piloter les moyens de pré-compensation de façon à gérer l'accordabilité en longueur d'onde du système;
- une carte détecteur 15 qui comprend pour chaque voie de détection un circuit analogique qui réalise notamment une adaptation d'impédance, un amplificateur, un convertisseur analogique numérique puis un composant logique programmable (par exemple un circuit FPGA) qui met en forme le signal.

Le micro-ordinateur 16 comprend également une carte d'acquisition numérique (non représentée) qui permet de traiter un flot de données numériques à fréquence variable et de l'afficher sur un écran au moyen d'une carte graphique (non représentée).

A titre d'exemple non limitatif, le traitement d'image mis en oeuvre dans la présente invention peut être une adaptation sans effort du traitement d'image tel que décrit notamment dans le document WO 2004/008952 et/ou le document WO 2004/010377.

En ce qui concerne le cas d'un guide d'image sans tête optique, le fonctionnement de l'appareillage est le même que celui décrit précédemment à l'exception de ce qui suit : en sortie du guide, la lumière divergente émergeant de la fibre injectée est diffusée dans l'échantillon et le signal de fluorescence est collecté dans un micro-volume situé entre la surface et une profondeur de quelques µm (selon le diamètre de coeur des fibres et leur ON). Grâce au balayage, l'échantillon est illuminé micro-volume par micro-volume. A chaque instant, le micro-volume excité dans le tissu émet alors un signal de fluorescence qui a la particularité d'être décalé vers des plus petites longueurs d'onde. Ce signal de fluorescence est capté par le guide d'image, puis suit le chemin inverse du faisceau d'excitation jusqu'au filtre dichroïque 6 qui va transmettre le signal de fluorescence vers la voie de détection. Les signaux détectés, les uns après les autres, sont notamment traités en temps réel grâce au même traitement d'image que celui décrit plus haut en référence à la figure 1 pour permettre la reconstruction d'une image en temps réel visualisée à l'écran.

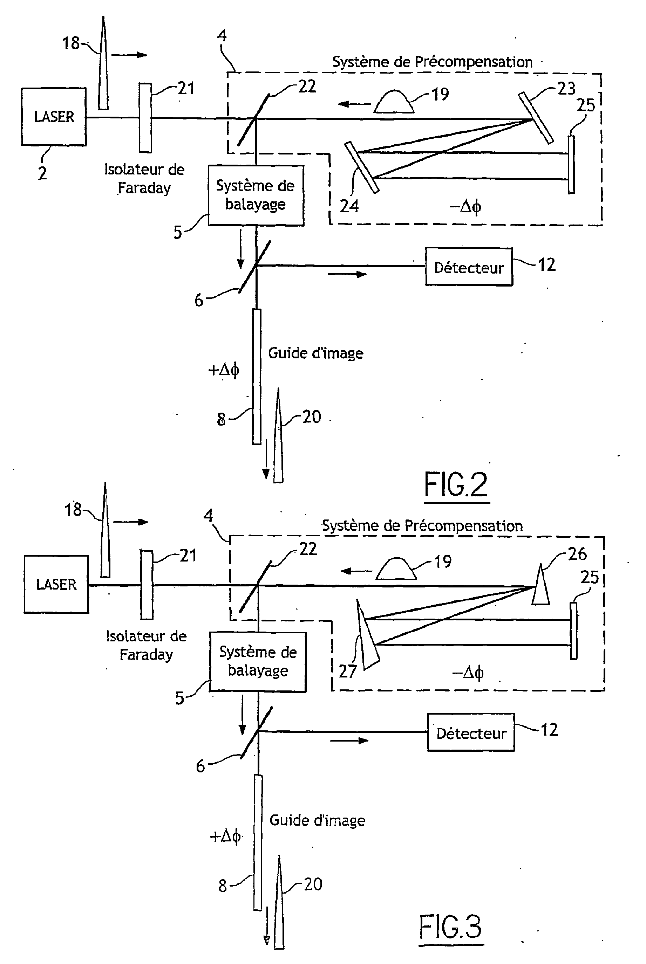

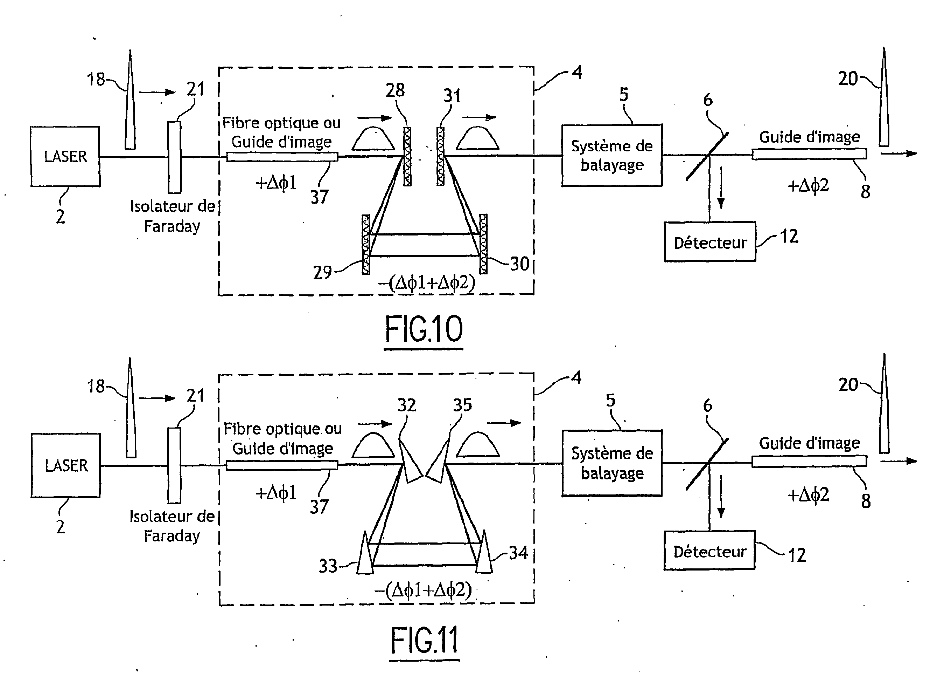

Les figures 2 à 14 (dont 8 à 11 et 14 selon l'invention) illustrent quelques exemples de dispositifs de pré-compensation. La pré compensation consiste à préparer l'impulsion laser ultra brève en lui conférant la largeur spectrale et la modulation de phase qui conduiront à sa compression temporelle optimale à la sortie du guide d'image 8. La technique employée a pour but de compenser la dispersion de vitesse de groupe de l'ensemble du système et de compenser également les effets non linéaires inévitables subis par l'impulsion lumineuse lors de sa propagation dans le guide d'image 8. Le principe de pré compensation envisagé est conforme à celui publié par S. W. Clark, F. O. Ildlay, and F. W. Wise, "Fiber delivery of femtosecond pulses from a Ti:sapphire laser", Optics Letters Vol. 26, NO. 17, september 1, 2001.

Typiquement, la pré-compensation selon l'invention comporte deux parties : un tronçon de fibre optique suivi par une ligne dispersive à réseaux de diffraction.
- le tronçon de fibre optique constitué d'une fibre optique unique : l'unique fibre optique utilisée est monomode pour la longueur d'onde laser. La longueur du tronçon est proche de celle du guide d'image 8 multi coeur. Cette longueur est optimisée en fonction des autres paramètres du système tels que par exemple la longueur du guide d'image, la longueur d'onde du faisceau laser, la puissance et la largeur des pulses en entrée et en sortie du guide d'image... Le diamètre du mode de cette fibre est supérieur au diamètre du mode des coeurs du guide d'image afin d'équilibrer les effets non linéaires rencontrés dans les deux parties de la pré-compensation. On utilise ainsi une fibre optique à large surface de mode (large mode area (LMA)) ou appartenant à la nouvelle génération de fibres optiques à gaine structurée air-silice. Ce tronçon de fibre monomode se caractérise par un certain taux de dispersion de vitesse de groupe normal (principalement à l'ordre 2 et 3) et par l'apparition d'effets non linéaires d'élargissement spectral (surtout sur les premiers millimètres du tronçon) qui sont contrôlés via le diamètre de mode. Ce tronçon, constitué d'une fibre optique unique, apporte un déphasage aux impulsions lasers excitatrices. Ce déphasage sert à la pré-compensation de la dispersion dans le guide d'image principal 8, mais il peut également servir à compenser toute autre dispersion introduite par le reste du système tel que la tête optique par exemple.
- la ligne dispersive à réseaux de diffraction : cette partie comporte deux réseaux de diffraction, fonctionnant en réflexion, plans, à forte efficacité, associés à un miroir plan de renvoi totalement réfléchissant. Les réseaux de diffraction se font face et sont disposés parallèlement à quelques centimètres l'un de l'autre. Le faisceau laser frappe successivement ces deux réseaux en incidence oblique avant d'atteindre le miroir plan qui renvoie la lumière approximativement sur elle-même. Le faisceau laser frappe ainsi quatre fois les réseaux avant de ressortir de la ligne dispersive. Le but de cette ligne dispersive est d'introduire un fort taux de dispersion de vitesse de groupe anormale dans le système. Ceci revient à retarder les photons les plus rouges du spectre laser ce qui correspond au comportement inverse de ce qui se passe à la fois dans le tronçon de fibre monomode cité ci-dessus mais également dans le guide d'image. Ce dispositif connu sous le nom de "ligne Treacy" est très utilisé dans les systèmes d'amplification à dérive de fréquence des chaînes lasers femtosecondes où il joue alors le rôle de compresseur d'impulsion en bout de chaîne. La ligne dispersive se caractérise par un certain taux de dispersion de vitesse de groupe anormale (principalement à l'ordre 2 et 3) dépendant du pas des réseaux, de la distance inter réseaux et de l'angle d'incidence sur ces réseaux.

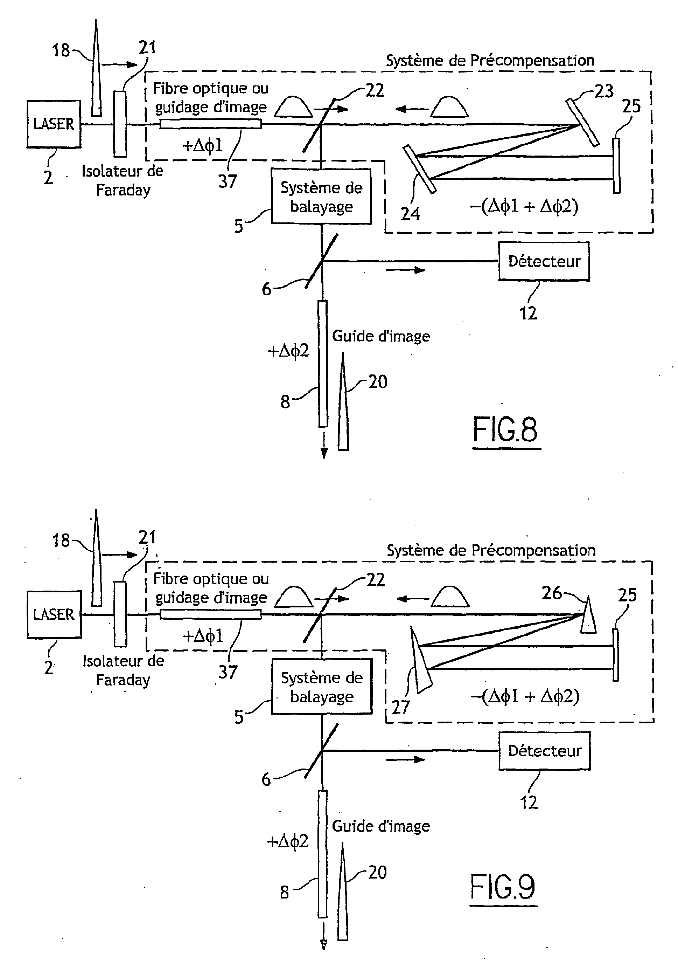

Les figures 2 à 14 sont des schémas simplifiés du système (dont les figures 8, 9, 10, 11, 14 selon l'invention) dans lequel le dispositif de pré-compensation 4 a été détaillé. Pour des raisons de clarté, le dispositif 3 n'apparaît pas. Les mêmes éléments de la figure 1 sont repris sur les figures 2 à 14 avec les mêmes références. On retrouve le laser 2, le système de balayage 5, le filtre dichroïque 6 qui transmet le faisceau excitateur vers l'échantillon et qui renvoie le signal de fluorescence vers le détecteur 12. Les figures 2 à 7 illustrent des dispositifs de compensation de base ne comportant pas un premier tronçon. Le faisceau sortant de l'isolateur de Faraday 21 se dirige directement vers une ligne dispersive à réseaux de diffraction ou à prismes. Sur la figure 2, cette ligne comporte deux réseaux de diffraction 23, 24 et un miroir 25. Le parcours du faisceau laser dans la ligne dispersive est le suivant : réflexion sur le premier réseau de diffraction 23 vers le second réseaux de diffraction 24, réflexion sur le second réseau de diffraction 24 vers le miroir 25 où il est totalement réfléchit vers le second réseau 24 puis le premier réseau 23. L'impulsion 19 sortant de la ligne dispersive est plus longue que celle 18 sortant du laser 2. Le faisceau
provenant du réseau de diffraction 23 réfléchit ensuite sur le miroir 22 en direction du système de balayage 5 puis vers le guide d'image 8. Les figures 2 à 14 sont des vues de dessus; en particulier sur les figures 2, 3, 6-9, le faisceau laser partant du laser 2 vers le réseau 23 passe au-dessus du miroir 22 sans le traverser. Par contre, le faisceau laser depuis le réseau 23 vers le système de balayage réfléchit sur le miroir 22. Ce miroir 22 peut être remplacé par un séparateur qui laisse passer le faisceau excitateur du laser vers la ligne dispersive et réfléchit le faisceau excitateur de la ligne dispersive vers le système de balayage, les deux faisceaux étant alors alignés. Mais, dans ce dernier cas, les pertes engendrées par les séparateurs sont importantes (seuls 25% du signal incident sont utilisés).

La dispersion linéaire et les effets non linéaires dans le guide d'image 8 modifient le profil temporel et spectral de l'impulsion d'excitation qui redevient sensiblement identique au profil 18 de l'impulsion sortant du laser 2. La ligne dispersive apporte un déphasage de -Δφ de façon à compenser approximativement le déphasage +Δφ apporté par le guide d'image 8. Sur la figure 3, les deux réseaux de diffraction sont remplacés par deux prismes 26 et 27, le parcours du faisceau laser excitateur est identique.

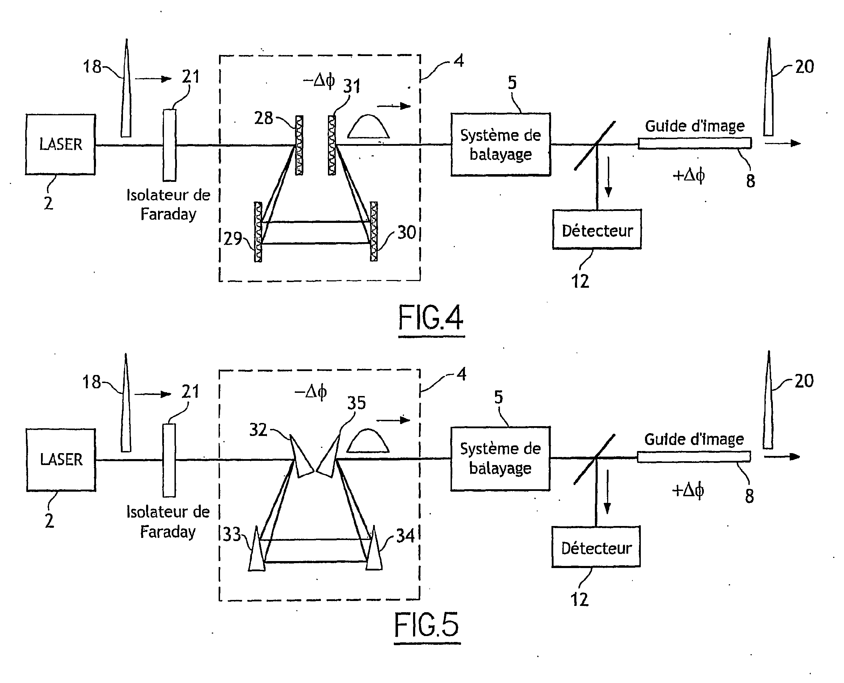

Sur les figures 4 et 5, la ligne dispersive et le miroir 22 sont remplacés par respectivement quatre réseaux de diffraction pour la figure 4 et quatre prismes pour la figure 5. Le faisceau laser excitateur se réfléchit successivement sur les quatre réseaux de diffraction 28, 29, 30 et 31 (prismes 32, 33, 34 et 35).

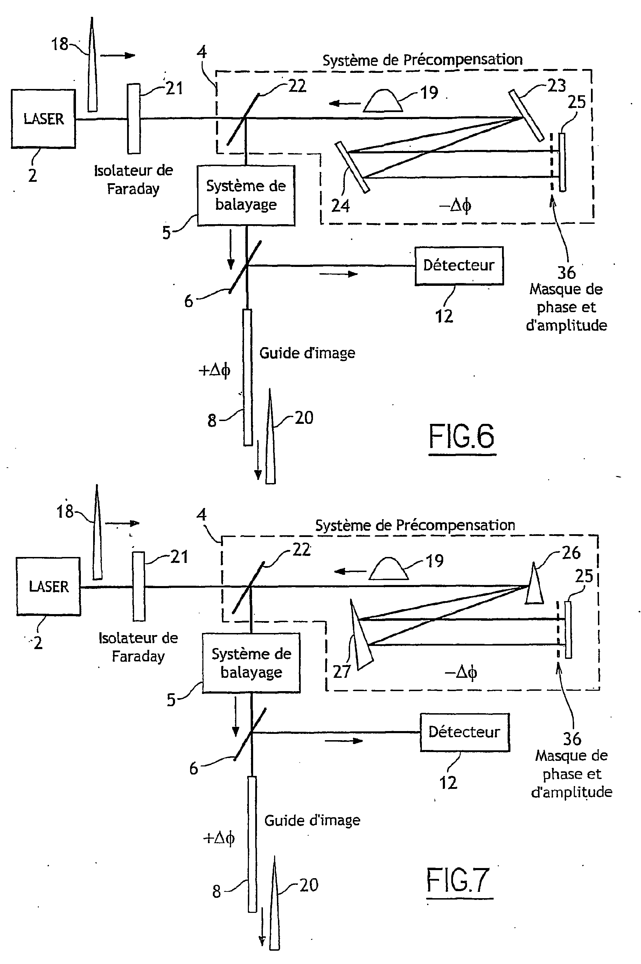

Les figures 6 et 7 correspondent aux figures 2 et 3 sur lesquelles un masque de phase et d'amplitude 36 a été introduit en amont du miroir 25. Ce masque permet d'améliorer les performances de la pré-compensation en ajustant précisément la forme du pulse par rapport à la dispersion du guide d'image. Il peut être constitué par l'assemblage de différentes lames de verres agissant sur la phase spectrale et d'un filtre variable de manière transversale et agissant sur l'amplitude spectrale.

Les figures 8 à 11 selon l'invention correspondent respectivement aux figures 2 à 5 mais avec en plus un tronçon 37 disposé soit en amont du miroir 22 (figures 2 et 3) soit en amont des quatre réseaux de diffraction (figure 4) ou des quatre prismes (figure 5).

Ce tronçon peut être constitué d'une fibre optique unique.

Cette fibre optique unique apporte un déphasage positif +Δφ1. Le guide d'image 8 apporte également un déphasage positif +Δφ2. Ainsi, la ligne dispersive (figures 8 à 11) apporte un déphasage négatif de -(Δφ1 + Δφ2).

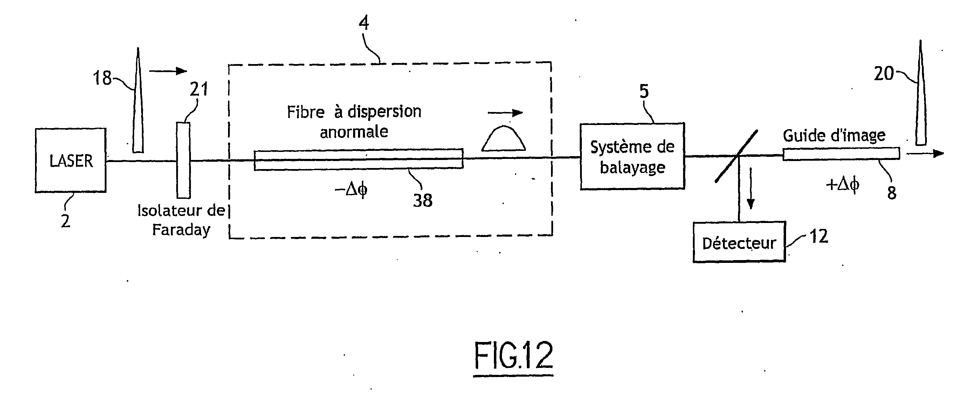

Comme on le voit sur la figure 12, l'étape de pré compensation peut aussi être obtenue à l'aide d'un unique tronçon 38 de fibre optique à dispersion anormale à la longueur d'onde laser. Cette fibre spécifique présente une dispersion nulle à des longueurs d'onde plus courtes que celle de la longueur d'onde laser. Ceci est obtenu par l'emploi d'un tronçon de fibre de nouvelle génération de longueur optimisée présentant l'une des structures suivantes : - fibre bi coeurs concentriques, fibre à gaine structurée air-silice, fibre photonique à coeur creux et à gaine structurée air silice, fibre photonique à coeur creux et à gaine de Bragg. Ce tronçon de fibre pré compensatrice se caractérise aussi par un diamètre de mode qui est optimisé afin de prendre en compte les effets non linéaires associés à la propagation dans ce guide d'onde.

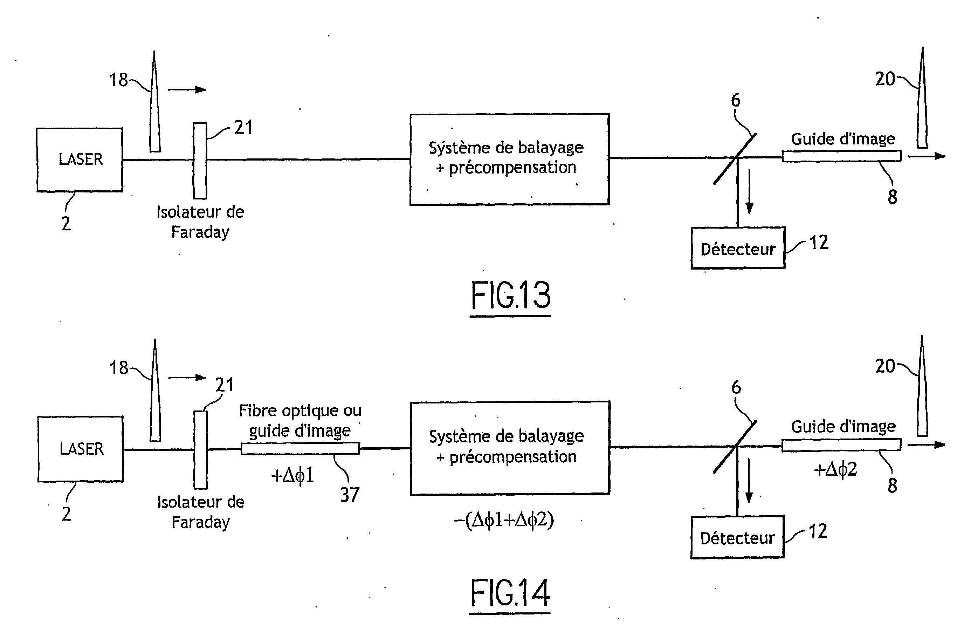

En variante de ce qui précède, la pré-compensation peut être intégrée dans le système de balayage comme on peut le voir sur les figures 13 et 14. En effet, l'un quelconque des dispositifs de compensation décrit sur les figures 2 à 11 peut être inséré sur un chemin optique dans le système de balayage 5.

Les avantages d'un microscope à base d'un guide d'image sont la compacité et la flexibilité, ce qui autorise une utilisation en endoscopie par insertion dudit guide d'image dans le tissu. Enfin, l'association de la microscopie multiphotonique avec une microscopie fibrée par guide d'image permet une acquisition d'image de fluorescence d'organe situé en profondeur de l'échantillon observé. Pratiquement, le système peut être de type adaptable, c'est à dire conçu sans la source laser et pouvant ainsi s'interfacer avec des sources lasers pré-existantes dans des laboratoires.

De nombreuses applications, en particulier là où on requiert des procédés non ou peu invasifs sont par exemple l'endoscopie urétrale lorsqu'une sonde optique avec un diamètre inférieur à 1mm est insérée dans une vessie par exemple; la colonoscopie du petit animal; la visualisation de la cornée et de la rétine; la visualisation des fibres musculaires et des nerfs; la microcirculation des leucocytes et du flux sanguin; l'architecture vasculaire et rénale; les membranes d'hépatocytes; et la neurobiologie in situ pour la visualisation des structures cérébrales profondes du petit animal vivant par exemple ou des applications cliniques potentielles pour l'homme.

## Revendications

1. Système d'imagerie multiphotonique fibré d'un échantillon (10), ce système comprenant :
- un laser pulsé (2) pour générer un faisceau laser d'excitation multiphotonique,
- un guide d'image (8) constitué d'une pluralité de fibres optiques, permettant d'illuminer l'échantillon (10) par un balayage point par point, et
- des moyens de balayage (5) pour diriger tour à tour le faisceau laser d'excitation dans une fibre du guide d'image (8),
le système étant **caractérisé en ce qu'**il comprend en outre :
- des moyens de compensation (4) pour compenser des effets de dispersion de vitesse de groupe et des effets non linéaires des impulsions d'excitation dans le guide d'image (8), ces moyens étant disposés entre le laser pulsé (2) et le guide d'image (8) la dispersion linéaire et les effets non linéaires dans le guide d'image (8) modifient le profil temporel et spectral de l'impulsion d'excitation qui redevient sensiblement identique au profil (18) de l'impulsion sortant du laser, de sorte que
les moyens de compensation comprenant un troncon de fibre optique suivi par une ligne dispersive, le tronçon de fibre optique étant constitué d'une unique fibre optique monomode, le diamètre du mode de cette fibre monomode étant supérieur au diamètre du mode des coeurs du guide d'image, ce tronçon de fibre étant agencé pour faire apparaître des effets non linéaires d'élargissement spectral, la ligne dispersive étant agencée pour introduire un taux de dispersion de vitesse de groupe anormale, en retardant les photons les plus rouges du spectre laser de sorte que l'impulsion (19) sortant de la ligne dispersive soit plus longue que celle (18) sortant du laser.

2. Système selon la revendication 1, **caractérisé en ce que** la ligne dispersive contient au moins deux prismes (26, 27 ; 32, 33, 34, 35).

3. Système selon la revendication 1, **caractérisé en ce que** la ligne dispersive contient au moins deux réseaux de diffraction (23, 24 ; 28, 29, 30, 31).

4. Système selon la revendication 1, **caractérisé en ce que** la ligne dispersive contient au moins deux prismes (26, 27 ; 32, 33, 34, 35), la fibre optique unique monomode (37) étant associée à la ligne dispersive de telle sorte que les déphasages introduits par cette fibre optique unique (37) et le guide d'image (8) sont compensés par le déphasage introduit par la ligne dispersive.

5. Système selon la revendication 1, **caractérisé en ce que** la ligne dispersive contient au moins deux réseaux de diffraction (23, 24 ; 28, 29, 30, 31), la fibre optique unique monomode (37) étant associée à la ligne dispersive de telle sorte que les déphasages introduits par cette fibre optique unique (37) et le guide d'image (8) sont compensés par le déphasage introduit par la ligne dispersive.

6. Système selon l'une quelconque des revendications 1 à 5, **caractérisé en ce que** la ligne dispersive est terminée par un miroir en amont duquel est disposé un masque de phase et d'amplitude (36).

7. Système selon l'une quelconque des revendications précédentes, **caractérisé en ce que** les moyens de compensation (4) sont intégrés dans les moyens de balayage (5).

8. Système selon l'une quelconque des revendications précédentes, **caractérisé en ce que** le laser pulsé (2) et les moyens de compensation (4) sont accordables en longueur d'onde.

9. Système selon l'une quelconque des revendications précédentes, **caractérisé en ce qu'**il comprend en outre des moyens d'injection disposés du côté proximal du guide d'image (8) et permettant de focaliser tour à tour le faisceau laser d'excitation dans une fibre donnée du guide d'image (8).

10. Système selon l'une quelconque des revendications précédentes, **caractérisé en ce qu'**il comprend en outre des premiers moyens de détection pour détecter un signal de fluorescence provenant de l'échantillon (10).

11. Système selon l'une quelconque des revendications précédentes, **caractérisé en ce qu'**il comprend en outre un filtre dichroïque apte à diriger les signaux en provenance de l'échantillon (10) vers des moyens de détection.

12. Système selon la revendication 11, **caractérisé en ce que** ledit filtre dichroïque est disposé entre les moyens de balayage (5) et le guide d'image (8).

13. Système selon l'une quelconque des revendications précédentes, **caractérisé en ce que** le laser pulsé (2) est un laser femtoseconde.

14. Système selon l'une quelconque des revendications 1 à 12, **caractérisé en ce que** le laser pulsé (2) est un laser picoseconde.

15. Système selon l'une quelconque des revendications précédentes, **caractérisé en ce que** le guide d'image (8) est composé d'une pluralité de fibres optiques monomodes ordonnées.

16. Système selon l'une quelconque des revendications précédentes, **caractérisé en ce qu'**il comprend une tête optique (9) pour focaliser le faisceau laser d'excitation sortant du guide d'image (8) dans l'échantillon (10).

17. Système selon l'une quelconque des revendications 1 à 15, **caractérisé en ce que** le guide d'image (8) est constitué de plusieurs milliers de fibres optiques et est sans tête optique, de sorte que les extrémités distales du guide d'image (8) soient agencées pour être placées à nue directement en contact de la surface de l'échantillon (10).

18. Procédé d'imagerie multiphotonique fibré d'un échantillon (10), dans lequel on génère un faisceau laser d'excitation multiphotonique par un laser pulsé (2), comprenant les étapes suivantes :
- on balaye l'échantillon (10) en dirigeant tour à tour le faisceau laser d'excitation dans une fibre d'un guide d'image (8) constitué d'une pluralité de fibres optiques,
- on illumine l'échantillon (10) par un balayage point par point à partir du faisceau laser d'excitation provenant du guide d'image (8), et
- on détecte un signal de fluorescence émis par l'échantillon (10)
**caractérisé en ce qu'**il comprend en outre les étapes suivantes :
- on fait passer le faisceau laser d'excitation par des moyens de compensation (4) disposés entre le laser pulsé (2) et le guide d'image (8) de manière à compenser des effets de dispersion de vitesse de groupe et des effets non linéaires des impulsions d'excitation dans le guide d'image (8), la dispersion linéaire et les effets non linéaires dans le guide d'image (8) modifiant le profil Temporel et spectral de l'impulsion d'excitation qui redevient sensiblement identique au profil (18) de l'impulsion sortant du laser, les moyens de compensation comprenant un tronçon de fibre optique suivi par une ligne dispersive, le tronçon de fibre optique étant constitué d'une unique fibre optique monomode, le diamètre du mode de cette fibre monomode étant supérieur au diamètre du mode des coeurs du guide d'image, ce tronçon de fibre faisant apparaître des effets non linéaires d'élargissement spectral, la ligne dispersive introduisant un taux de dispersion de vitesse de groupe anormale, en retardant les photons les plus rouges du spectre laser de sorte que l'impulsion (19) sortant de la ligne dispersive soit plus longue que celle (18) sortant du laser.

19. Procédé selon la revendication 18, **caractérisé en ce qu'**on détecte l'ensemble du signal de fluorescence sortant du guide d'image (8).

## Claims

1. System for carrying out fibre-type multiphoton imaging of a sample (10), this system comprising:
- a pulsed laser (2) for generating a multiphoton excitation laser beam,
- an image guide (8) constituted by a plurality of optical fibres and allowing the sample (10) to be illuminated by a point-by-point scanning,
- scanning means (5) for directing in turn the excitation laser beam into a fibre of the image guide (8),
**characterized in that** it also comprises:
- compensation means (4) for compensating for non linear effects and group velocity dispersion effects of the excitation pulses in the image guide (8), these means being disposed between the pulsed laser (2) and the image guide (8), so that linear dispersion and non-linear effects in the image guide (8) modify the temporal and spectral profile of the excitation pulse which returns to being approximately identical to the profile (18) of the pulse leaving the laser,
the compensation means comprising a section of optical fibre followed by a dispersive line, the section of optical fibre being constituted by a single optical fibre which is single-mode, this single-mode optical fibre comprising a mode diameter greater than the mode diameter of the cores of the image guide , this section of fibre being arranged for intoducing non-linear effects of spectral broadening, the dispersive line being arranged for introducing a rate of abnormal group velocity dispersion by delaying the most red photons of the laser spectrum so that the pulse (19) leaving the dispersive line is longer than that (18) leaving the laser.

2. System according to claim 1, **characterized in that** the dispersive line contains at least two prisms (26, 27; 32, 33, 34, 35).

3. System according to claim 1, **characterized in that** the dispersive line contains at least two diffraction gratings (23, 24; 28, 29, 30, 31).

4. System according to claim 1, **characterized in that** the dispersive line contains at least two prisms (26, 27; 32, 33, 34, 35), the single-mode optical fibre (37) being associated with the dispersive line so that the phase shifts introduced by this single optical fibre (37) and the image guide (8) are compensated for by the phase shift introduced by the dispersive line.

5. System according to claim 1, **characterized in that** the dispersive line contains at least two diffraction gratings (23, 24; 28, 29, 30, 31), the single-mode optical fibre (37) being associated with the dispersive line so that the phase shifts introduced by this single optical fibre (37) and the image guide (8) are compensated for by the phase shift introduced by the dispersive line.

6. System according to any one of claims 1 to 5, **characterized in that** the dispersive line ends with a mirror upstream of which a phase and amplitude mask is arranged.

7. System according to any one of the preceding claims, **characterized in that** the compensation means (4) are integrated into the scanning means (5).

8. System according to any one of the preceding claims, **characterized in that** the pulsed laser (2) and the compensation means (4) are tunable as regards wavelength.

9. System according to any one of the preceding claims, **characterized in that** it also comprises injection means arranged on the proximal side of the image guide (8) and making it possible to focus in turn the excitation laser beam into a given fibre of the image guide (8).

10. System according to any one of the preceding claims, **characterized in that** it also comprises first detection means for detecting a fluorescence signal originating from the sample (10).

11. System according to any one of the preceding claims, **characterized in that** it also comprises a dichroic filter which is able to direct the signals originating from the sample (10) towards detection means.

12. System according to claim 11, **characterized in that** said dichroic filter is arranged between the scanning means (5) and the image guide (8).

13. System according to any one of the preceding claims, **characterized in that** the pulsed laser (2) is a femtosecond laser.

14. System according to any one of claims 1 to 12, **characterized in that** the pulsed laser (2) is a picosecond laser.

15. System according to any one of the preceding claims, **characterized in that** the image guide (8) is composed of a plurality of sequenced single-mode optical fibres.

16. System according to any one of the preceding claims, **characterized in that** it comprises an optical head (9) for focussing the excitation laser beam leaving the image guide (8) into the sample (10).

17. System according to any one of claims 1 to 15, **characterized in that** the image guide (8) is constituted by several thousands of optical fibres and is without optical head, so that the distal ends of the image guide (8) are intended to be placed bare directly in contact with the surface of the sample (10).

18. Method for carrying out fibre-type multiphoton imaging of a sample (10), in which a multiphoton excitation laser beam is generated by a pulsed laser (2), comprising the following steps:
- the sample (10) is scanned by directing in turn the excitation laser beam into a fibre of an image guide (8) constituted by a plurality of optical fibres,
- the sample (10) is illuminated by a point-by-point scanning from the excitation laser beam originating from the image guide (8), and
- a fluorescence signal emitted by the sample (10) is detected
**characterized in that** it also comprises the following steps:
- the excitation laser beam is passed through compensation means (4) disposed between the pulsed laser (2) and the image guide (8) in order to compensate for non linear effects and group velocity dispersion effects of the excitation pulses in the image guide (8), linear dispersion and non-linear effects in the image guide (8) modifying the temporal and spectral profile of the excitation pulse which returns to being approximately identical to the profile (18) of the pulse leaving the laser, the compensation means comprising a section of optical fibre followed by a dispersive line, the section of optical fibre being constituted by a single optical fibre which is single-mode, this single-mode optical fibre comprising a mode diameter greater than the mode diameter of the cores of the image guide, this section of fibre intoducing non-linear effects of spectral broadening, the dispersive line introducing an rate of abnormal group velocity dispersion by delaying the most red photons of the laser spectrum so that the pulse (19) leaving the dispersive line is longer than that (18) leaving the laser.

19. Method according to claim 18, **characterized in that** the entire fluorescence signal leaving the image guide (8) is detected.

## Patentansprüche

1. System zur faseroptischen Multiphotonen-Abbildung einer Probe (10), wobei dieses System umfasst:
- einen Pulslaser (2) zum Erzeugen eines Multiphotonen-Anregungslaserstrahls,
- einen Bildleiter (8), der aus einer Mehrzahl von Lichtleitfasern besteht und gestattet, die Probe (10) durch eine punktweise Abtastung auszuleuchten, und
- Abtastmittel (5), um den Anregungslaserstrahl der Reihe nach in eine Faser des Bildleiters (8) zu richten,
wobei das System **dadurch gekennzeichnet ist, dass** es ferner aufweist:
- Kompensationsmittel (4) zum Kompensieren von Dispersionseffekten der Gruppengeschwindigkeit und von nichtlinearen Effekten der Anregungsimpulse im Bildleiter (8), wobei diese Mittel zwischen dem Pulslaser (2) und dem Bildleiter (8) angeordnet sind, so dass die lineare Dispersion und die nichtlinearen Effekte in dem Bildleiter (8) das zeitliche und spektrale Profil des Anregungsimpulses verändern, das im wesentlichen wieder identisch zum Profil (18) des aus dem Laser tretenden Impulses wird, wobei die Kompensationsmittel einen Lichtleitfaserabschnitt, gefolgt von einer dispersiven Leitung aufweisen, wobei der Lichtleitfaserabschnitt aus einer einzigen Monomode-Lichtleitfaser besteht, wobei der Durchmesser der Mode dieser Monomode-Faser größer ist als der Durchmesser der Mode der Kerne des Bildleiters, wobei dieser Faserabschnitt dazu vorgesehen ist, die nichtlinearen Effekte spektraler Erweiterung erscheinen zu lassen, wobei die dispersive Leitung dazu vorgesehen ist, einen Dispersionsgrad anormaler Gruppengeschwindigkeit einzuleiten, indem die weiter zum Roten hin liegenden Photonen des Laserspektrums verzögert werden, so dass der aus der dispersiven Leitung (19) austretende Impuls (19) länger als derjenige (18) ist, der aus dem Laser austritt.

2. System nach Anspruch 1, **dadurch gekennzeichnet, dass** die dispersive Leitung zumindest zwei Prismen (26, 27; 32, 33, 34, 35) umfasst.

3. System nach Anspruch 1, **dadurch gekennzeichnet, dass** die dispersive Leitung zumindest zwei Beugungsgitter (23, 24; 28, 29, 30, 31) umfasst.

4. System nach Anspruch 1, **dadurch gekennzeichnet, dass** die dispersive Leitung zumindest zwei Prismen (26, 27; 32, 33, 34, 35) umfasst, wobei die einzige Monomode-Lichtleitfaser (37) derart der dispersiven Leitung zugeordnet ist, dass die von dieser einzigen Lichtleitfaser (37) und dem Bildleiter (8) hervorgerufenen Phasenänderungen durch die von der dispersiven Leitung hervorgerufenen Phasenänderung kompensiert werden.

5. System nach Anspruch 1, **dadurch gekennzeichnet, dass** die Streulinie zumindest zwei Beugungsgitter (23, 24; 28, 29, 30, 31) umfasst, wobei die einzige Monomode-Lichtleitfaser (37) derart der dispersiven Leitung zugeordnet ist, dass die von dieser einzigen Lichtleitfaser (37) und dem Bildleiter (8) hervorgerufenen Phasenänderungen durch die von der dispersiven Leitung hervorgerufenen Phasenänderung kompensiert werden.

6. System nach einem der Ansprüche 1 bis 5, **dadurch gekennzeichnet, dass** die dispersive Leitung von einem Spiegel abgeschlossen wird, dem eine Phasen- und Amplitudenmaske (36) vorgelagert ist.

7. System nach einem der vorangehenden Ansprüche, **dadurch gekennzeichnet, dass** die Kompensationsmittel (4) in den Abtastmitteln (5) integriert sind.

8. System nach einem der vorangehenden Ansprüche, **dadurch gekennzeichnet, dass** der Pulslaser (2) und die Kompensationsmittel (4) in der Wellenlänge abstimmbar sind.

9. System nach einem der vorangehenden Ansprüche, **dadurch gekennzeichnet, dass** es ferner Einkopplungsmittel aufweist, die auf der proximalen Seite des Bildleiters (8) angeordnet sind und gestatten, den Anregungslaserstrahl der Reihe nach in eine gegebenen Faser des Bildleiters (8) zu fokussieren.

10. System nach einem der vorangehenden Ansprüche, **dadurch gekennzeichnet, dass** es ferner erste Erfassungsmittel zum Erfassen eines Fluoreszenzsignals aufweist, das von der Probe (10) stammt.

11. System nach einem der vorangehenden Ansprüche, **dadurch gekennzeichnet, dass** es ferner einen dichroitischen Filter aufweist, der die von der Probe (10) stammenden Signale zu den Erfassungsmitteln richten kann.

12. System nach dem Anspruch 11, **dadurch gekennzeichnet, dass** der dichroitische Filter zwischen den Abtastmitteln (5) und dem Bildleiter (8) angeordnet ist.

13. System nach einem der vorangehenden Ansprüche, **dadurch gekennzeichnet, dass** der Pulslaser (2) ein Femtosekundenlaser ist.

14. System nach einem der Ansprüche 1 bis 12, **dadurch gekennzeichnet, dass** der Pulslaser (2) ein Pikosekundenlaser ist.

15. System nach einem der vorangehenden Ansprüche, **dadurch gekennzeichnet, dass** der Bildleiter (8) aus einer Mehrzahl von geordneten Monomode-Lichtleitfasern zusammengesetzt ist.

16. System nach einem der vorangehenden Ansprüche, **dadurch gekennzeichnet, dass** es einen optischen Kopf (9) zum Fokussieren des aus dem Bildleiter (8) austretenden Anregungslaserstrahls in die Probe (10) ist.

17. System nach einem der Ansprüche 1 bis 15, **dadurch gekennzeichnet, dass** der Bildleiter (8) aus mehreren Tausend Lichtleitfasern besteht und ohne optischen Kopf ist, so dass die distalen Enden des Bildleiters (8) dazu vorgesehen sind, blank in unmittelbarem Kontakt mit der Oberfläche der Probe (10) gebracht zu werden.

18. Verfahren zur faseroptischen Multiphotonen-Abbildung einer Probe (10), wobei ein Multiphotonen-Anregungslaserstrahl durch einen Pulslaser (2) erzeugt wird, welches Verfahren die nachfolgenden Schritte umfasst:
- Abtasten der Probe (10), indem der Anregungslaserstrahl der Reihe nach in eine Faser eines Bildleiters (8) gerichtet wird, der aus einer Mehrzahl von Lichtleitfasern besteht,
- Ausleuchten der Probe (10) durch punktweise Abtastung ausgehend von dem Anregungslaserstrahl, der von dem Bildleiter (8) stammt, und
- Erfassen eines Fluoreszenzsignals, das von der Probe (10) emittiert wird, **dadurch gekennzeichnet, dass** es ferner die nachfolgenden Schritte umfasst:
- Leiten des Anregungslaserstrahls durch Kompensationsmittel (4), die zwischen Pulslaser (2) und Bildleiter (8) angeordnet sind, so dass Dispersionseffekte der Gruppengeschwindigkeit und nichtlineare Effekte der Anregungsimpulse in dem Bildleiter (8) kompensiert werden, wobei die lineare Dispersion und die nichtlinearen Effekte in dem Bildleiter (8) das zeitliche und spektrale Profil des Anregungsimpulses verändern, das im wesentlichen wieder identisch zum Profil (18) des aus dem Laser austretenden Impulses wird, wobei die Kompensationsmittel einen Lichtleitfaserabschnitt, gefolgt von einer dispersiven Leitung aufweisen, wobei der Lichtleitfaserabschnitt aus einer einzigen Monomode-Lichtleitfaser besteht, wobei der Durchmesser der Mode dieser Monomode-Faser größer ist als der Durchmesser der Mode der Kerne des Bildleiters, wobei dieser Faserabschnitt die nichtlinearen Effekte spektraler Erweiterung erscheinen lässt, wobei die dispersive Leitung einen Dispersionsgrad anormaler Gruppengeschwindigkeit einleitet, indem die weiter zum Roten hin liegenden Photonen des Laserspektrums verzögert werden, so dass der aus der dispersiven Leitung austretende Impuls (19) länger als derjenige (18) ist, der aus dem Laser austritt.

19. Verfahren nach Anspruch 18, **dadurch gekennzeichnet, dass** das gesamte aus dem Bildleiter (8) austretende Fluoreszenzsignal erfasst wird.
